# EUROPEAN PATENT APPLICATION

(11) **EP 4 652 918 A1**
(43) Date of publication of application: **26.11.2025**
(21) Application number: 25178010.2
(22) Date of filing: 21.05.2025
(51) Int. Cl.: A61B 3/113, G02B 27/01, G06F 3/00, G06F 3/01, G06V 40/18

(54) **ESTIMATING PRESCRIPTION GLASSES STRENGTH FOR HEAD-MOUNTED DISPLAY USERS**

(30) Priority: 23.05.2024 SE 2450552; 23.05.2024 SE 2450553
(71) Applicant: Tobii AB, 182 17 Danderyd (SE)
(72) Inventor: Selin, Mårten, 182 17 Danderyd (SE); Le, Huu, 182 17 Danderyd (SE); Rana, Pravin Kumar, 182 17 Danderyd (SE)

(57) **Abstract**

A method for estimating the strength of prescription glasses for a user of a head-mounted display is disclosed. The method involves illuminating a user's eyes and prescription glasses using illuminators, capturing images of the eyes, the prescription glasses, and reflections on the prescription glasses using at least one camera, and determining the power of lenses of the prescription glasses by analyzing the reflections. The method can be used to improve the user experience in virtual reality, augmented reality, and mixed reality applications by accounting for the user's prescription glasses.

## Description

### Field

The technology relates to the field of head-mounted displays (HMD), specifically for virtual reality (VR), augmented reality (AR), and mixed reality (MR) applications. It focuses on eye tracking systems and methods for estimating the strength of prescription glasses worn by users of these head-mounted displays.

### Background

Eye tracking technology has become increasingly important in various fields, including virtual reality (VR), augmented reality (AR), and mixed reality (MR) applications. These applications often involve the use of wearable devices containing displays, also known as head-mounted displays (HMDs), that provide users with an immersive experience. HMDs are well known and are typically utilized in VR and AR systems. In these systems the displays are used to provide a user with an experience that simulates either a different reality in the case of VR, or an enhanced reality in the case of AR. Eye tracking systems integrated into HMDs enable various functionalities, such as gaze-based interaction, user attention analysis, and improved visual rendering. U.S. Patent Application Publication Number 2017/0090564 describes a wearable HMD incorporating an eye-tracking system to detect gaze direction of a user.

A typical eye tracking system comprises one or more cameras and illuminators that capture images of the user's eyes. The system then employs a mathematical model and computer analysis to determine the gaze direction by identifying various eye features, such as the pupil and corneal reflections, in the captured images. The accuracy of the eye tracking system is crucial for providing a seamless and immersive experience to the user.

However, a significant portion of the population requires prescription glasses to correct their vision. The presence of prescription glasses between the user's eyes and the eye tracking system introduces distortion in the acquired eye images. This distortion affects the sizes, shapes and locations of eye features, such as the pupil and corneal reflections, leading to impaired eye tracking performance. The degree of distortion primarily depends on the prescription of the glasses, e.g. on the lens power. The shape and material of the lenses of the glasses determines the prescription.

The prior art has attempted to address the issue of distortion caused by prescription glasses in eye tracking systems. For example, US10342425B1 describes using illuminators to detect user gaze based on glints from the user's pupils. This disclosure describes a technique for identifying reflections that are caused by the user's prescription glasses and compensating for them to ensure accurate gaze direction can be determined.

However, the prior art does not provide a comprehensive solution to the problem of impaired eye tracking performance for users with prescription glasses. Although the distortion introduced by prescription glasses is manageable when the parameters of the glasses are known, the distortion affects the eye tracking system's accuracy in analyzing eye features and mapping world features when the parameters of the glasses are not known.

### Summary

According to a first aspect of the disclosure, a method is provided for estimating the strength of prescription glasses for a user of a head-mounted display. This method comprises illuminating a user's eyes and prescription glasses using illuminators, capturing images of the eyes, the prescription glasses, and reflections on the prescription glasses using at least one camera, and determining the power of lenses of the prescription glasses by analyzing the reflections. This method allows for a non-invasive and efficient way to estimate the strength of user's prescription glasses, providing for a more accurate eye tracking system. The mapping from image coordinates to world coordinates becomes more accurate. This enhanced accuracy facilitates, for example, a more accurate estimation of cornea position based on undistorted glints and pupil triangulation enabling us to achieve more accurate gaze mapping as well as entrance pupil position (EPP) estimates. Consequently, this leads to improved eye tracking performance and an enhanced user experience.

Optionally in some examples, the method further comprises setting camera brightness controlling parameters of the at least one camera to capture the reflections on the prescription glasses. The camera brightness controlling parameters comprise at least one of exposure time or camera gain. This feature allows for better image quality and more accurate reflection capture, which can lead to more precise estimation of the prescription glasses' strength.

Optionally in some examples, the method further involves determining positions of the reflections in the images and identifying illuminators responsible for each of the reflections. This feature can help in accurately determining the power of the lenses by providing more data points for analysis.

According to another aspect of the disclosure, the method for determining the power of the lenses comprises using a correlation between the power of the lenses and a radius of curvature of a surface of lenses. The radius of curvature of the lenses is estimated based on the positions of the reflections in the images and the positions of the respective illuminators. This approach allows for a more direct and accurate estimation of the lens power.

Optionally in some examples, the reflections used to estimate the radius of curvature of the lenses are reflections on an outer surface of the lenses. This feature can simplify the estimation process and reduce potential errors, as the outer surface is more accessible and easier to image.

Optionally in some examples, the correlation used in determining the power of the lenses is a base curve rule. This can provide a reliable and accurate estimation of the lens power.

Optionally in some examples, the method further comprises approximating the surface of the lenses as a spherical shape. This approximation can simplify the calculation process and is often accurate enough for most practical applications.

Optionally in some examples, the method further comprises determining power for each of a right lens and a left lens in a pair of the prescription glasses. This feature allows for a more personalized and accurate estimation of the prescription glasses' strength, as it takes into account the potential differences in power between the two lenses.

According to another aspect of the disclosure, the method further comprises estimating the curvature of an outer surface and/or an inner surface of the prescription glasses for each of the lenses by utilizing image processing of the captured images. This feature can significantly improve the accuracy and efficiency of the estimation process.

Optionally in some examples, the method comprises utilizing a pre-trained regressor to estimate the curvature of an outer surface and/or an inner surface of the prescription glasses. The pre-trained regressor can leverage machine learning techniques to make more accurate predictions based on the captured images. The pre-trained regressor may thus estimate the curvature by a machine learning process. This feature allows for continuous improvement of the estimation accuracy over time, as the machine learning model can learn from new data and refine its predictions.

Optionally in some examples, the machine learning process comprises at least one of linear regression and/or non-linear regression. These regression techniques can provide a robust and flexible framework for estimating the lens power, as they can model a wide range of relationships between the lens curvature and power.

Optionally in some examples, the pre-trained regressor is trained using annotated features comprising curvatures and reflections from the outer surface and/or the inner surface of the lens from images of users wearing prescription glasses. This provides for enhancing the accuracy of the regressor, as it can learn from real-world data that accurately reflects the variations in lens curvature and reflections.

According to another aspect of the disclosure, a head-mounted display is provided, which comprises an eye tracking system configured to determine a gaze direction, and an entrance pupil location of a user's eyes, at least one camera configured to capture images of the user's eyes and the prescription glasses, illuminators configured to illuminate the user's eyes and the prescription glasses, and a computer analysis system configured to analyze the captured images and determine power of lenses of the prescription glasses by analyzing reflections on the prescription glasses. This head-mounted display can provide a comprehensive solution for estimating the strength of a user's prescription glasses, which can significantly enhance the user experience in virtual reality applications.

Optionally in some examples, the eye tracking system of the head-mounted display is configured to perform pupil center cornea reflection (PCCR) eye tracking. This feature can provide a more accurate and reliable estimation of the user's gaze direction, which can be for many virtual reality applications.

Optionally in some examples, the illuminators of the head-mounted display are LED illuminators having a wavelength range of 750nm - 1400nm. This feature can provide a suitable illumination for capturing high-quality images of the user's eyes and prescription glasses, which can lead to more accurate estimation of the prescription glasses' strength.

Optionally in some examples, the computer analysis system of the head-mounted display is configured to optimize camera brightness parameters comprising at least one of exposure time or camera gain to capture reflections on an outer surface and/or an inner surface of the prescription glasses. This feature can enhance the image quality and improve the accuracy of the reflection capture, which can lead to a more precise estimation of the lens power.

Optionally in some examples, the computer analysis system of the head-mounted display is configured to identify illuminators responsible for each reflection and determine a curvature of the lens by analyzing the reflections. This feature can provide more data points for the analysis and improve the accuracy of the lens power estimation.

Optionally in some examples, the computer analysis system of the head-mounted display is configured to approximate a radius of curvature and estimate power of the lenses based on the curvature. This feature can simplify the calculation process and provide a more direct and accurate estimation of the lens power.

Optionally in some examples, the computer analysis system of the head-mounted display is configured to utilize a pre-trained regressor to estimate the curvature of the outer surface and/or the inner surface of the prescription glasses for each of the lenses. This feature can leverage machine learning techniques to improve the accuracy and efficiency of the lens power estimation.

According to another aspect of the disclosure, a method is provided for estimating the strength of prescription glasses for a user of a head-mounted display. This method comprises capturing images of each of the user's eyes and the prescription glasses from different viewpoints, determining a number of candidate ellipses representing the same iris from the captured images, determining a center and radius of each iris in a 3D space using triangulation of the candidate ellipses, and analyzing a deviation in the determined radius of each iris to infer the power of the lenses of the prescription glasses. An alternative method to estimate the strength of a user's prescription glasses is thereby provided, allowing for an accurate eye tracking system. An improved eye tracking performance and an enhanced user experience is provided.

Optionally in some examples, the method for determining each ellipse comprises employing edge detection to fit an ellipse for each iris. This feature can provide a robust and accurate way to represent the iris, which can improve the accuracy of the lens power estimation.

Optionally in some examples, the method for determining each ellipse comprises utilizing an ellipse regressor. This feature can leverage machine learning techniques to provide a more accurate and efficient way to represent the iris, which can lead to a more precise estimation of the lens power.

Optionally in some examples, the deviation in the radius of each iris is the estimated difference in size of the radius from a human average iris radius. This feature can provide a more direct and intuitive way to infer the lens power, as it directly relates the lens power to a measurable physical characteristic of the user's eyes.

Optionally in some examples, the human average iris radius is 5.5mm. This feature provides a suitable reference point for the deviation in the radius of each iris, which can improve the accuracy of the lens power estimation.

Optionally in some examples, determining the power of the lenses is based on a pre-calculated relationship between the power of the lenses of the prescription glasses and the deviation in the determined radius of an iris. This feature can provide a more direct and accurate estimation of the lens power, as it leverages the relationship between the lens power and the iris radius deviation.

Optionally in some examples, the pre-calculated relationship is determined through a machine learning process where the estimated iris radius is known prior to refraction through the lenses. This feature can enhance the accuracy of the lens power estimation, as the machine learning process can learn from previous data and refine its predictions.

Optionally in some examples, the machine learning process comprises a regression model. This feature can provide a robust and flexible framework for estimating the lens power, as it can model a wide range of relationships between the iris radius deviation and the lens power.

According to another aspect of the disclosure, a head-mounted display is provided, which comprises an eye tracking system configured to determine a gaze direction of a user's eyes, at least one camera configured to capture images of each of the user's eyes and the prescription glasses from different viewpoints, and a computer analysis system configured to determine a number of candidate ellipses representing the same iris from the captured images, determine a center and radius of each iris in a 3D space using triangulation of the candidate ellipses, and analyze a deviation in the determined radius of each iris to infer the power of the lenses of the prescription glasses. This head-mounted display can provide a comprehensive solution for estimating the strength of a user's prescription glasses, which can significantly enhance the eye tracking performance.

Optionally in some examples, the computer analysis system of the head-mounted display is further configured to estimate a difference in size of the radius from a human average iris radius. This feature can provide a more direct and intuitive way to infer the lens power, as it directly relates the lens power to a measurable physical characteristic of the user's eyes.

Optionally in some examples, the computer analysis system of the head-mounted display is further configured to determine the power of the lenses of prescription glasses based on a pre-calculated relationship between the power of the lenses of the prescription glasses and the deviation in the determined radius of each iris. This feature can provide a more direct and accurate estimation of the lens power, as it leverages a pre-established relationship between the lens power and the iris radius deviation.

Optionally in some examples, the pre-calculated relationship is determined through a machine learning process where the estimated iris radius is known prior to refraction through lenses. This feature can enhance the accuracy of the lens power estimation, as the machine learning process can learn from previous data and refine its predictions.

Optionally in some examples, the machine learning process comprises a regression model. This feature can provide a robust and flexible framework for estimating the lens power, as it can model a wide range of relationships between the iris radius deviation and the lens power.

### Brief Description of the Drawings

Examples are described in more detail below with reference to the appended drawings.
Figure 1 is a schematic illustration of a head-mounted display and an eye tracking system according to an example of the disclosure.
Figure 2 is a schematic view of an eye tracking system including a plurality of illuminators, with each illuminator located at a respective fixed position in relation to a user's eye.
Figure 3a shows example eye images of users without prescription glasses.
Figure 3b shows example eye images of users with prescription glasses.
Figure 4 displays example reflections on the glasses' outer surface and detected reflections on the glasses' outer surface.
Figure 5 is a schematic illustration of a user's eye at a distance from a lens of prescription glasses.
Figure 6 is a schematic illustration of a lens of prescription glasses having a curvature.
Figure 7a is a diagram of the geometry of an eye tracking system comprising a camera and two illuminators according to an example of the disclosure.
Figure 7b demonstrates the sufficiency of a known geometry and the detection of two illuminator reflections for reconstructing the base curve which corresponds to the curvature of the outer surface of the lens.
Figure 8a shows a grouping of prescribed lens powers (Rx) upon common base curves.
Figure 8b displays Tscherning's ellipse.
Figure 9a-d illustrates inner and outer surface curvatures of the prescription glasses worn by users in eye images for training the curvature regressor based on associated annotated metadata.
Figure 10 is an iris triangulation visualization for the left eye, where r is the radius of an estimated "virtual" iris in 3D.
Figure 11 shows triangulated iris radius with different glasses diopters using an eye model with a known iris radius of ~2.5mm.

### Detailed Description

The detailed description set forth below provides information and examples of the disclosed technology with sufficient detail to enable those skilled in the art to practice the disclosure.

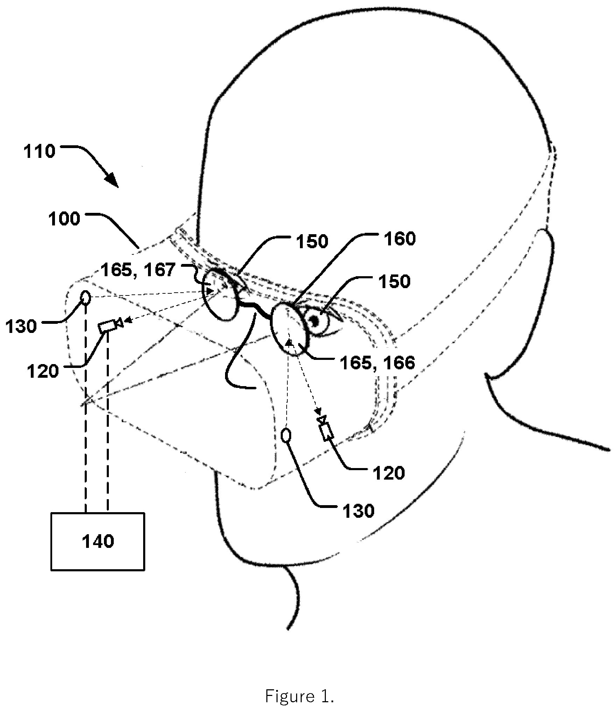

Figure 1 shows a schematic illustration of a head-mounted display 100 and an eye tracking system 110 according to an example of the disclosure. The head-mounted display 100 may be a VR headset or other wearable device utilizing VR, AR, or MX systems and applications, which is placed on the user's head for viewing of VR, AR, or MX content. The head-mounted display 100 comprises an eye tracking system 110, at least one camera 120, illuminators 130, and a computer analysis system 140. The head-mounted display 100 is configured to provide a virtual, augmented, or mixed reality experience for the user. The eye tracking system 110 is designed to determine the gaze direction and entrance pupil location of the user's eyes 150. The camera 120 is configured to capture images 190 of the user's eyes 150 and the prescription glasses 160. The illuminators 130 are configured to illuminate the user's eyes 150 and the prescription glasses 160. The computer analysis system 140 is configured to analyze the captured images 190 and determine the power of lenses 165 of the prescription glasses 160 by analyzing reflections 180 on the prescription glasses 160. This provides for a more accurate eye tracking system 110. The mapping from image coordinates to world coordinates becomes more accurate. This enhanced accuracy facilitates, for example, a more accurate estimation of cornea position based on undistorted glints and pupil triangulation enabling us to achieve more accurate gaze mapping as well as accurate entrance pupil position (EPP) estimates. Consequently, this leads to improved eye tracking performance and an enhanced user experience. The power of the lenses 165 may be determined by using a correlation between the power and a radius of curvature 170 of a surface of lenses 165, as described further below, where the radius of curvature 170 of the lenses 165 may be estimated based on the positions of the reflections 180 in the images 190 and the positions of the respective illuminators 130.

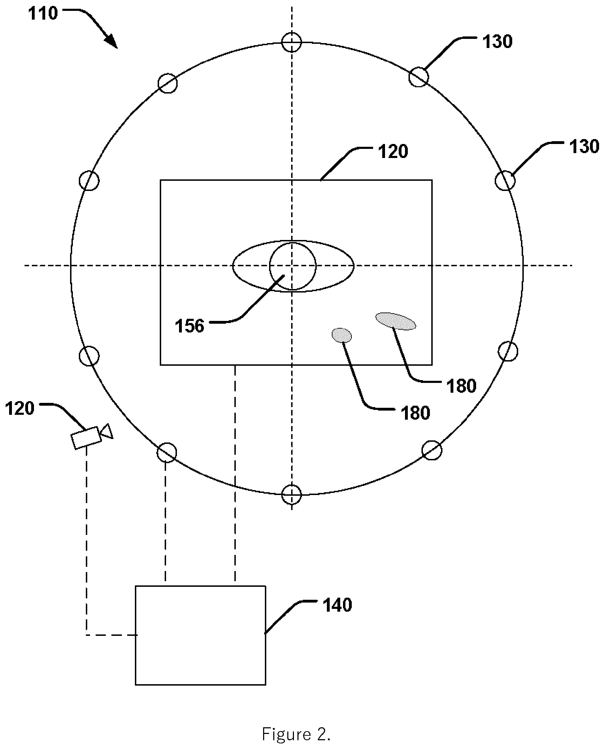

Figure 2 shows a further schematic view of an eye tracking system 110 according to an example. The eye tracking system 110 comprises a plurality of illuminators 130. Each illuminator 130 of the plurality of illuminators 130 is located at a respective fixed position in relation to an eye of a user, illustrated by iris 156, when the system user is using a head-mounted display 100 provided with the eye tracking system 110. Specifically, the plurality of illuminators 130 may be arranged along the periphery of a substantially circle outline, which may, for example, be the periphery of one of the VR lenses in the head-mounted display 100. The reflections 180 from the prescription glasses 160 are shown. The computer analysis system 140 is in communication with one or more cameras 120 and the illuminators 130 and is configured to analyze the reflections 180 and determine the power of the lenses 165 of the prescription glasses 160, as described in relation to figure 1. In one example the computer analysis system 140 is configured to analyze the reflections 180 as well as the reflections on the cornea, i.e. the glints in the user's eyes, to determine the power of the lenses 165.

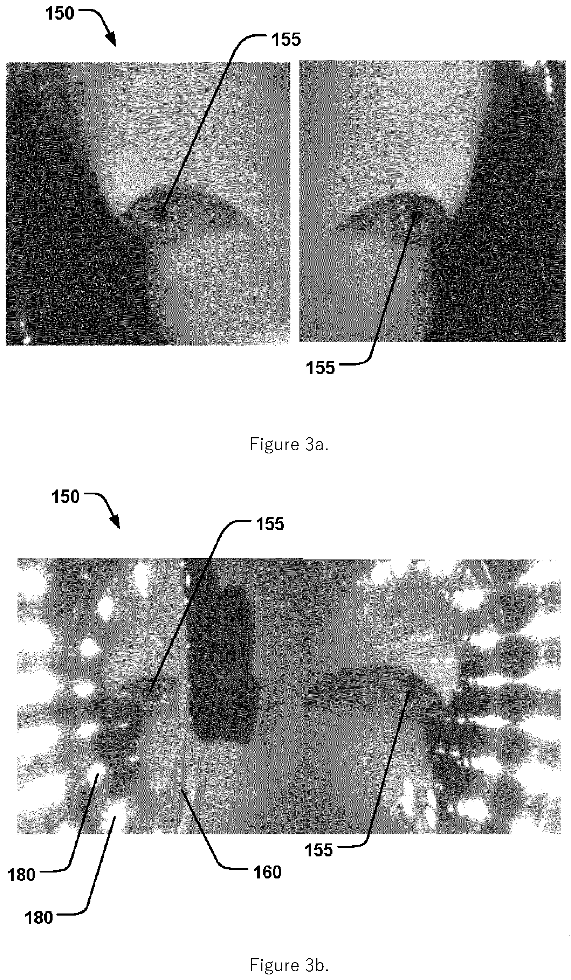

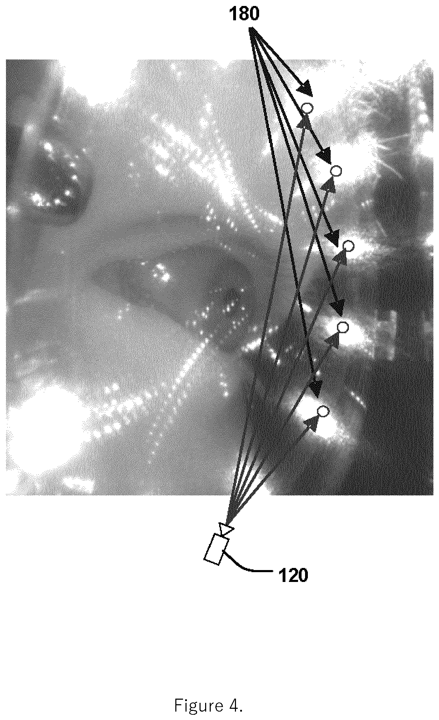

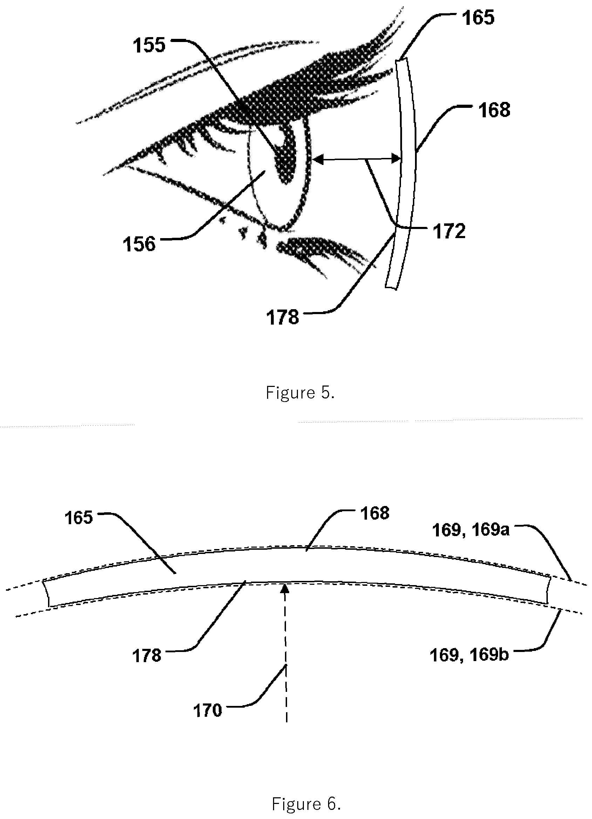

Figure 3a shows example eye images of users without prescription glasses 160. Glints are shown in the eyes of the user. Figure 3b shows example eye images of users with prescription glasses 160 and reflections 180 on a surface thereof. The prescription glasses 160 introduces distortion in the acquired eye images. This distortion affects the sizes, shapes and locations of eye features, such as the pupil and corneal reflections, which would affect eye tracking performance in the prior art. Figure 4 displays example reflections 180 on the glasses' 160 outer surface and corresponding detection of these reflections 180 by a camera 120 of a head-mounted display 100 according to an example of the present disclosure. At least two reflections on the glasses' outer surface may be identified. Figure 5 is a schematic illustration of a user's eye and iris 156 at a distance 172 from a lens 165 of prescription glasses 160. The lens 165 has an outer surface 168 and an inner surface 178, where the inner surface face the user's eye.

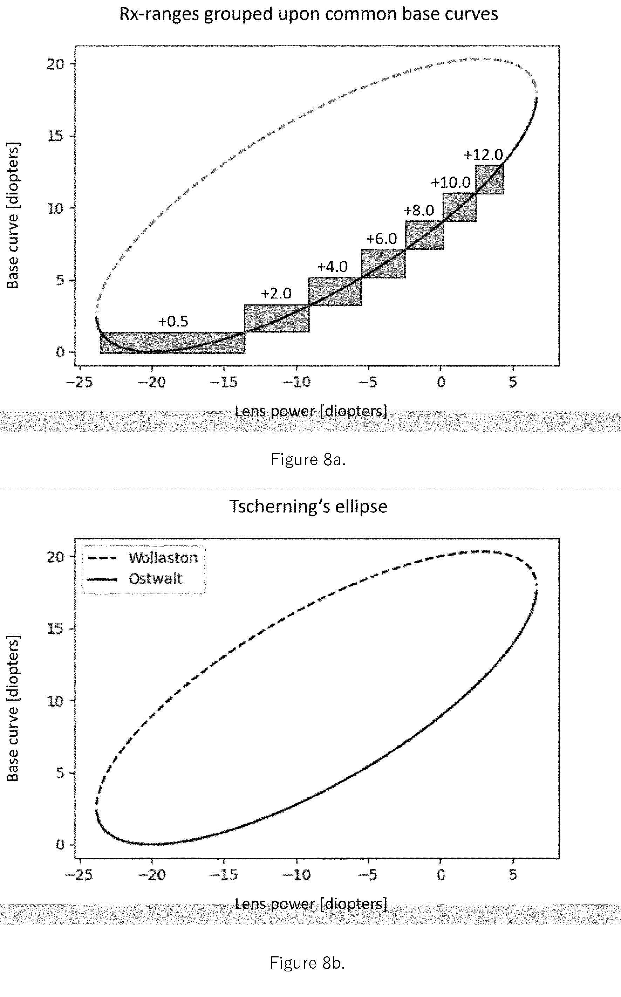

Figure 6 is a schematic illustration of a lens 165 of prescription glasses 160 having a curvature 169. The curvature of the outer surface 168 is denoted 169a. The curvature of the outer surface 168 of the lenses 165 is typically referred to as the base curve for prescription glasses 160. Traditionally, the base curve is assumed to be spherical, following a universal design rule. The curvature of the inner surface 178 is denoted 169b. The inner surface 178 is typically referred to as the Rx surface in the field, and is where the prescription is implemented. The corresponding radius of curvature 170 is illustrated. The power of the lens 165 is estimated based on the curvature 169, 169a, 169b, or the corresponding radius of curvature 170. The power of the lens 165 can be determined by utilizing the correlation as illustrated in figures 8a-b.

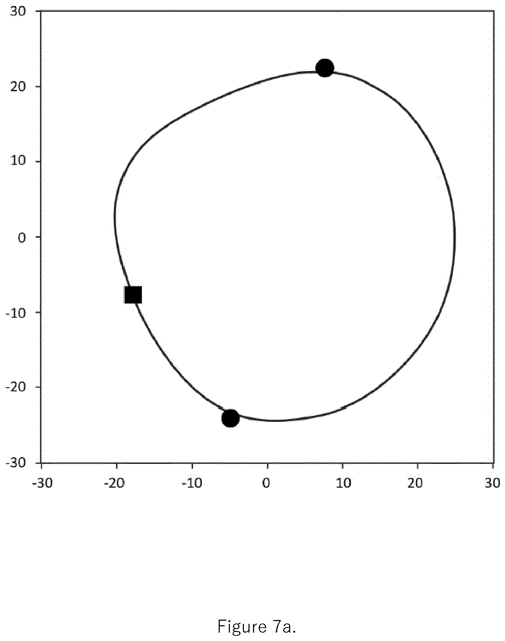

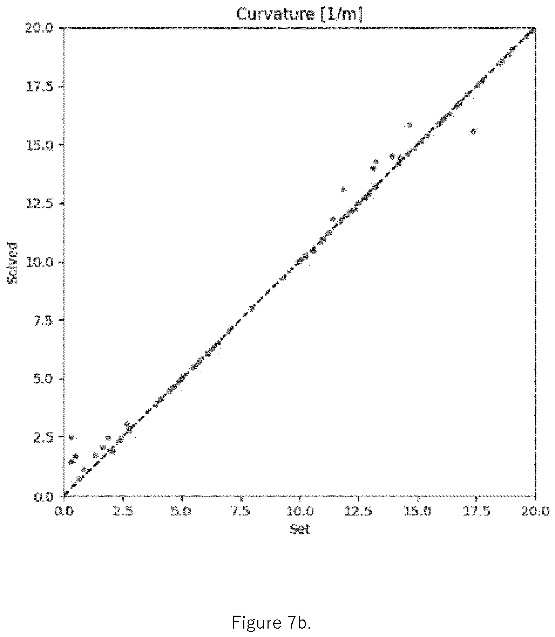

Figure 7a is a diagram of the geometry of an eye tracking system 110, comprising one camera (square) and two illuminators (circles) positioned at the periphery of a VR lens (solid line), according to an example of the disclosure, with x- and y-axes indicating distances in mm. Figure 7b is an example diagram to illustrate that for 100 randomized glasses positions and outer surface curvatures, the method according to the invention, accurately reconstructed the base-curve, i.e. the curvature of the outer surface 168 of the glasses. Here, an optimization method was employed to demonstrate the sufficiency of a known geometry (camera and illuminator locations) and the detection of two illuminator reflections for reconstructing the base curve.

Figure 8a shows a grouping of prescribed lens powers (Rx) upon common base curves. Figure 8b displays Tscherning's ellipse, which is a graphical representation of the outer surface curvatures (base curves) that minimizes astigmatism for different lens powers. Tscherning's ellipse showcases its solutions in two branches, the steeper Wollaston branch and the flatter Ostwalt branch. Since the Wollaston branch yields impractically steep lens surfaces, the Ostwalt branch is always used in modern optical design. The Ostwalt branch of the Tscherning's ellipse is referred to as an example of a base curve rule. By employing a base curve rule, the radius of curvature can be approximated and assigned a power to the prescription lenses. The optical design principles delineate how the base curve should correspond to the desired lens power in order to minimize lens aberrations. This principle is known as the aforementioned Tscherning's Ellipse or some simplification thereof (e.g.,"Vogel's rule"), known to a skilled person. While additional design considerations, aesthetic factors or preferences of the optometrist or the subject may influence the curvature, the deviation from the Ostwalt branch of Tscherning's ellipse must be small lest the lens should yield significant astigmatism. Therefore, by measuring the base curve of the prescription glasses, reasonably accurate estimate of its lens power can be obtained.

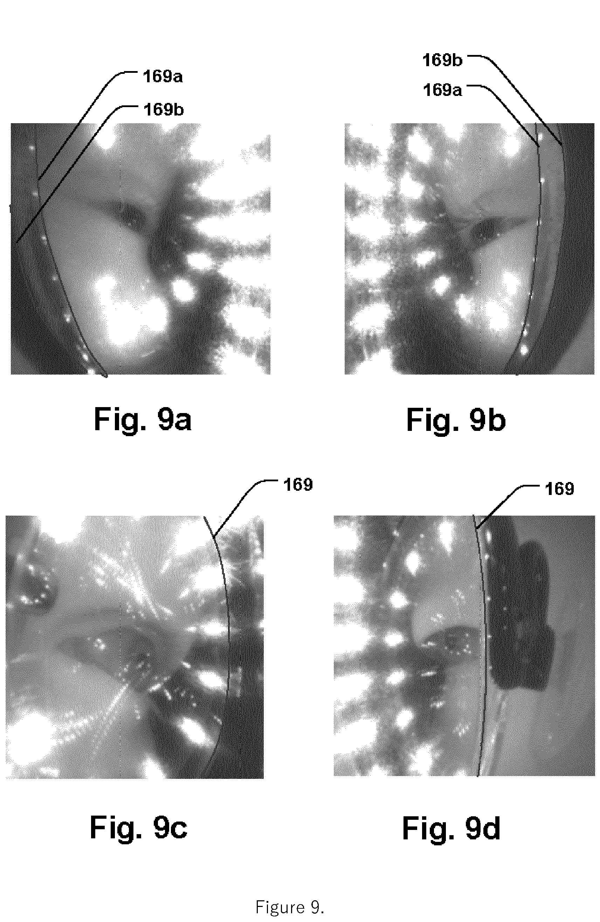

Figures 9a-d illustrate different examples of eye images 190 of users wearing prescription glasses. The curvature 169, 169a, 169b, of the outer surface 168 and/or an inner surface 178 of the prescription glasses 160 for each of the lenses 165 may be estimated by utilizing image processing of the captured images 190. In a further method for estimating the strength of prescription glasses 160 for a user of a head-mounted display 100 a curvature regressor is trained based on annotated features comprising curvatures 169, 169a, 169b, and reflections 180 from the outer surface 168 and/or the inner surface 178 of the lens from the images 190. Annotation metadata in the images 190 with particular patterns of reflections 180 may thus specify respective diopter values and the radius of curvature 170, which is utilized to train the curvature regressor. The lens power may then be determined by utilizing the correlation between the estimated radius of curvature 170, of the outer surface 168 and/or the inner surface 178, and the lens power, e.g as illustrated in figures 8a-b. The computer analysis system 140 may thus be configured to utilize the pre-trained regressor to estimate the curvature 169, 169a, 169b, of the outer surface 168 and/or the inner surface 178 to estimate the power of the lenses 165. An improved eye tracking performance may thus be provided.

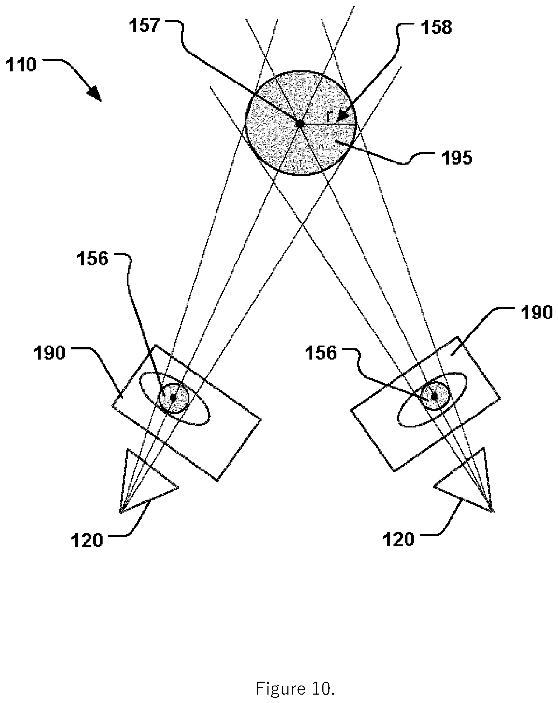

Figure 10 is an iris triangulation visualization for a left eye, where r is the radius of an estimated "virtual" iris 195 in 3D. A similar method would be performed for a right eye, however, Figure 10 shows an example for only one eye. This figure demonstrates the process of determining the center 157 and radius 158 of each iris 156 in a 3D space using triangulation of the candidate ellipses. The 3D space is typically represented by an x-, y-, z-coordinate system of a space surrounding the user of the head-mounted display 100. Two images 190 are captured for each of the left and right eyes 150 of a user wearing an XR headset, e.g. by utilizing dual-camera 120 eye tracking solution. Computer analysis may be performed on the acquired eye images 190 to detect the presence of prescription glasses 160 on the user's eyes 150. If it is detected that glasses 160 are positioned in front of the user's eyes 150, then for each eye image 190, the method is used to determine the position of the iris 156. This can be accomplished through either; i) utilizing edge detection and employing a RANSAC-based method to fit an ellipse, or ii) employing an iris-based ellipse regressor to determine an ellipse or a similar method known in the art. For each eye (left or right), once the two ellipses representing the same iris 156 are obtained from the cameras, the center 157 and radius 158 is calculated of each iris in 3D, i.e. of the determined virtual iris 195. The resulting values will represent the "virtual" estimated iris positions, accounting for the refraction caused by the glasses 160. Typically, human irises have an average radius of approximately 5.5mm. However, when using triangulation to compute the 3D iris radius, this value may change because of the glasses' lenses.

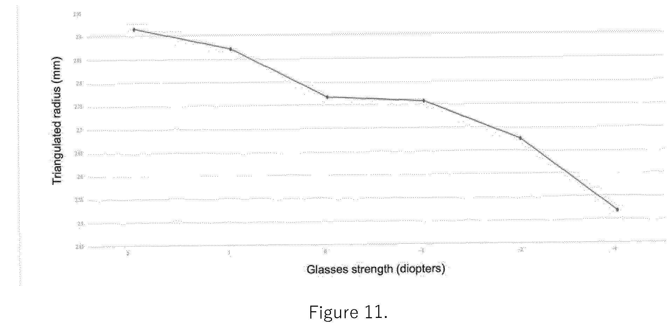

By analyzing the deviation in the triangulated iris radius 158, the strength of the glasses may be determined. The more negative the diopters, the smaller the triangulated radius, see Figure 11. To approximate the deviation accurately, data may be collected with eye models representing a physical eye, where the exact iris radius 158 is known prior to refraction through glasses 160 or train a model where the real human eye iris radius is annotated with and without prescription glasses with known strength. By establishing a relationship between the glasses' strength and the deviation in the computed radius through a learning process such as linear regression and reinforcement learning process, the strength of the glasses 160 can be effectively inferred. Figure 11 shows triangulated iris radius 158 with different glasses diopters using a model of an eye with a known iris radius of ~2.5mm.

The disclosure comprises several components that work together to estimate the strength of prescription glasses 160 for a user of a head-mounted display 100. These components comprise a head-mounted display 100, an eye tracking system 110, a camera 120, illuminators 130 and a computer analysis system 140.

In some implementations, the head-mounted display 100 is designed to provide a virtual, augmented, or mixed reality experience for the user. The head-mounted display 100 comprises an eye tracking system 110, at least one camera 120, illuminators 130, and a computer analysis system 140. The head-mounted display 100 is configured to be worn by a user and is designed to track the user's eye movements and gaze direction. The head-mounted display 100 may be used in various applications, including but not limited to, gaming, training simulations, virtual meetings, and other interactive experiences.

In one example, the eye tracking system 110 is integrated into the head-mounted display 100. The eye tracking system 110 is designed to determine the gaze direction and entrance pupil location of the user's eyes 150. The eye tracking system 110 may use various techniques and technologies to track the user's eye movements and gaze direction. The eye tracking system 110 may comprise one or more cameras 120 and illuminators 130. The eye tracking system 110 may also comprise a computer analysis system 140 that is configured to analyze the captured images 190 and determine the power of lenses 165 of the prescription glasses 160 by analyzing reflections 180 on the prescription glasses 160. The eye tracking system 110 of the head-mounted display 100 may be configured to perform pupil center cornea reflection (PCCR) eye tracking. PCCR is a well-known and readily understood method of determining a user's gaze. Further information on this method can be found in multiple places, including Guestrin, E. D., Eizenman, E., "General theory of remote gaze estimation using the pupil center and corneal reflections," Biomedical Engineering, IEEE Transactions, vol. 53, no. 6, pp. 1124, 1133, June 2006.

The camera 120 is configured to capture images 190 of the user's eyes 150, the prescription glasses 160 and the reflections 180 created by the illuminators 130. The camera 120 may be a digital camera, a video camera, or any other type of image capturing device. The camera 120 may be positioned in various locations on the head-mounted display 100 to capture images 190 of the user's eyes 150 and the prescription glasses 160 from different angles or viewpoints. The camera 120 may be configured to capture images 190 at a high frame rate, such as 60-120 frames per second, to accurately track the user's eye movements and gaze direction.

The images 190 may comprise various features, such as the user's eyes 150, the prescription glasses 160, and reflections 180 on the prescription glasses 160. The images 190 may be captured at a high resolution, such as 1920x1080 pixels, to provide detailed information about the user's eyes 150 and the prescription glasses 160. The images 190 may be analyzed by the computer analysis system 140 to determine the power of the lenses 165 of the prescription glasses 160. The images 190 may be stored in a memory of the head-mounted display 100 or transmitted to an external device for further analysis.

In some configurations, the process involves setting the camera brightness controlling parameters for optimal reflection capture. The camera brightness controlling parameters may comprise the exposure time and the camera gain. The exposure time is the length of time that the camera's shutter is open, allowing light to reach the image sensor. The camera gain is the amplification of the signal from the image sensor. By adjusting the camera parameters, the process can optimize the capture of the reflections 180 on the prescription glasses 160, improving the accuracy of the power estimation.

In one implementation, the process begins by illuminating the user's eyes 150 and prescription glasses 160 using the illuminators 130. The illuminators 130 emit light that is reflected by the lenses 165 of the prescription glasses 160, creating reflections 180 that can be captured by the camera 120. The illuminators 130 are thus configured to provide illumination for the user's eyes 150 and the prescription glasses 160. The illuminators 130 may be positioned in various locations on the head-mounted display 100 to illuminate the user's eyes 150 and the prescription glasses 160 from different angles. The illuminators 130 may be of various types, such as LED illuminators, VCSELs or fiber optic illuminators providing near-infrared light. The illuminators 130 may emit light at various intensities and wavelengths. For instance, the illuminators 130 may emit light with an intensity of 500-1000 lumens and a wavelength range of 750nm - 1400nm. The illuminators 130 may be configured to project light through the lenses 165 of the prescription glasses 160 onto the user's pupil 155. The light emitted by the illuminators 130 may be reflected by the lenses 165 of the prescription glasses 160 and captured by the camera 120 as reflections 180.

The computer analysis system 140 is configured to analyze the images 190 captured by the camera 120. The computer analysis system 140 may comprise one or more processors and memory for storing instructions and data. The computer analysis system 140 may be configured to perform various tasks, such as identifying the user's eyes 150 and the prescription glasses 160 in the images 190, determining the positions of the reflections 180 in the images 190, identifying the illuminators 130 responsible for each reflection 180, determining the curvature 169 of the lenses 165 by analyzing the reflections 180, approximating the radius of curvature 170 of the lenses 165, and estimating the power of the lenses 165 based on the radius of curvature 170. The computer analysis system 140 may use various algorithms and machine learning techniques to perform these tasks.

In one example, the reflections 180 are due to emitted light from the illuminators 130 which is reflected by the lenses 165 of the prescription glasses 160. The reflections 180 may be captured by the camera 120 and included in the images 190. The reflections 180 may provide information about the curvature 169 and the radius of curvature 170 of the lenses 165. The reflections 180 may be analyzed by the computer analysis system 140 to determine the power of the lenses 165. The positions of the reflections 180 in the images 190 may be used to estimate the radius of curvature 170 of the lenses 165. The positions of the reflections 180 may also be used to identify the illuminators 130 responsible for each reflection 180. The analysis of the reflections 180 may be based on various assumptions, such as the assumption that the outer surface 168 of the lenses 165 follows a spherical shape, and the assumption that the distance 172 between the surface of the eye's cornea and the lenses 165 is constant.

In some configurations, the user of the head-mounted display 100 wears prescription glasses 160. The prescription glasses 160 comprise lenses 165 that correct the user's vision. The lenses 165 may have various powers, which are determined by the user's prescription for eye sight correction and position of wear.

The disclosure specifies a method for estimating the strength of prescription glasses 160 for a user of a head-mounted display 100. The method comprises illuminating the user's eyes and prescription glasses 160, capturing images 190 of the eyes 150, the prescription glasses 160, and reflections 180 on the prescription glasses, and determining the power of the lenses 165 of the prescription glasses 160 by analyzing the reflections 180.

In one example, the method begins by illuminating the user's eyes 150 and prescription glasses 160. This illumination may be provided by one or more illuminators 130, which are part of the eye tracking system 110. The illuminators 130 may emit light at various intensities and wavelengths to illuminate the user's eyes 150 and the prescription glasses 160. The illuminators 130 may be positioned in various locations on the head-mounted display 100 to illuminate the user's eyes 150 and the prescription glasses 160 from different angles or viewpoints. The light emitted by the illuminators 130 may be reflected by the lenses 165 of the prescription glasses 160 and captured by the camera 120 as reflections 180.

The illuminators 130 provide the necessary light for the camera 120 to capture clear images 190 of the user's eyes 150 and the prescription glasses 160. The light emitted by the illuminators 130 is reflected by the lenses 165 of the prescription glasses 160, creating reflections 180 that can be analyzed to determine the power of the lenses 165. The illuminators 130 may be controlled to emit light at different intensities, depending on the requirements of the specific application.

In one configuration, the method involves capturing images 190 of the user's eyes 150, the prescription glasses 160, and the reflections 180 on the prescription glasses 160. The images 190 are captured using a camera 120, which is part of the eye tracking system 110. The camera 120 may be positioned in various locations on the head-mounted display 100 to capture images 190 of the user's eyes 150 and the prescription glasses 160 from different viewpoints. The camera 120 may be configured to capture images 190 at a high frame rate, such as 60-120 frames per second, to accurately track the user's eye movements and gaze direction.

In some examples, the method involves determining the positions of the reflections 180 in the images 190. The reflections 180 are light emitted by the illuminators 130 and reflected by the lenses 165 of the prescription glasses 160. The positions of the reflections 180 in the images 190 provide information about the curvature 169 and the radius of curvature 170 of the lenses 165. The positions of the reflections 180 may be determined using various image processing techniques, such as edge detection and pattern recognition.

In one implementation, the method involves identifying the illuminators 130 responsible for each reflection 180. The illuminators 130 are part of the eye tracking system 110 and are configured to emit light that is reflected by the lenses 165 of the prescription glasses 160. By identifying the illuminators 130 responsible for each reflection 180, the method can determine the positions of the reflections 180 in the images 190 and estimate the radius of curvature 170 of the lenses 165.

In some configurations, the method comprises determining the power of the lenses 165 of the prescription glasses 160 by analyzing the reflections 180. The reflections 180 are light emitted by the illuminators 130 and reflected by the lenses 165 of the prescription glasses 160. The reflections 180 provide information about the curvature 169 and the radius of curvature 170 of the lenses 165. By analyzing the reflections 180, the method can estimate the power of the lenses 165. The analysis of the reflections 180 may be based on various assumptions, such as the assumption that the outer surface 168 of the lenses 165 follows a spherical shape, and the assumption that the distance 172 between the surface of the eye's cornea and the lenses 165 is constant. The analysis of the reflections 180 may be performed using various algorithms and machine learning techniques.

In some examples, the method involves using the base curve rule or a curvature regressor to determine the power of the lenses 165 of the prescription glasses 160. The base curve rule and the curvature regressor are techniques for estimating the power of the lenses 165 based on the curvature 169 and the radius of curvature 170 of the lenses 165. The base curve rule and the curvature regressor may be used in combination with other techniques, such as image processing and machine learning, to accurately estimate the power of the lenses 165.

The base curve rule is a technique for estimating the power of the lenses 165 based on the curvature 169 and the radius of curvature 170 of the lenses 165. The method involves using a correlation between the power of the lenses 165 and the radius of curvature 170 of the lenses 165 to estimate the power of the lenses 165. The correlation may be based on the base curve rule, such as illustrated in figures 8a-b. By using this correlation, the method can estimate the power of the lenses 165.

In some implementations, the application of the base curve rule involves various assumptions and specifications. For instance, it may be assumed that the outer surface 168 of the lenses 165 follows a spherical shape. This assumption simplifies the analysis of the reflections 180 and the estimation of the power of the lenses 165. It may also be assumed that the distance 172 between the surface of the eye's cornea and the lenses 165 is constant.

In one example, the method involves using a curvature regressor to estimate the strength of the prescription glasses 160. The curvature regressor is a machine learning model that is trained to estimate the curvature 169 of the lenses 165 based on the reflections 180 in the images 190. The curvature regressor may be trained using a dataset that comprises images 190 of users' eyes 150 and prescription glasses 160 (see. e.g. figures 9a-d of examples of such real-world data), along with annotations that specify the curvature 169 and the power of the lenses 165. The images 190 may be acquired by an eye tracking camera such as a camera 120 imaging the user wearing prescription glasses 160. The known curvatures and lens power values are thus associated with image features such how the reflections 180 are shown in the images 190, and a relationship between the known values and the image features can be established. The curvature regressor can thus be trained on recognizing these relationships. The pre-trained regressor is thus capable of estimating the curvature 169 of the lenses 165 based on the reflections 180 in the images 190. By using a curvature regressor, the method can accurately estimate the power of the lenses 165 based on the reflections 180 in the images 190.

In one example, the power of the lenses 165 of the prescription glasses 160 is determined through an analysis of the iris 156 of the user's eyes 150. The process involves capturing multiple images 190 of each of the user's eyes 150 and the prescription glasses 160 from different viewpoints, such as illustrated in the example of figure 10. From these images 190, a number of candidate ellipses representing the same iris 156 are determined. The center 157 and radius 158 of each iris 156 in a 3D space are then determined using triangulation of the candidate ellipses. A deviation in the determined radius 158 of each iris 156 is analyzed to infer the power of the lenses 165 of the prescription glasses 160.

In some implementations, the computer analysis system 140 uses a machine learning process to analyze the images 190 and determine the power of the lenses 165. The machine learning process may comprise various steps, such as training a model, testing the model, and using the model to make predictions. The model may be trained using a dataset that comprises images 190 of users' eyes 150 and prescription glasses 160, along with annotations that specify the power of the lenses 165. The model may be tested using a separate dataset that comprises images 190 of users' eyes 150 and prescription glasses 160, along with annotations that specify the power of the lenses 165. The trained model is then used to estimate the power of the lenses 165 based on the images 190 of the user's eyes 150 and the prescription glasses 160. The machine learning process may use various algorithms and techniques, such as linear regression, non-linear regression, decision trees, neural networks, and deep learning. The machine learning process may be implemented using various software tools and libraries, such as TensorFlow, PyTorch, and scikit-learn. The disclosure has potential applications in various fields, including virtual reality, augmented reality, and mixed reality.

In one example, the disclosure is used in a virtual reality system. The virtual reality system comprises a head-mounted display 100 that provides a virtual reality experience for the user. The head-mounted display 100 comprises an eye tracking system 110 that tracks the user's eye movements and gaze direction. The eye tracking system 110 uses the disclosure to estimate the strength of the user's prescription glasses 160, improving the accuracy of the eye tracking and enhancing the user's virtual reality experience.

In another example, the disclosure is used in an augmented reality system. The augmented reality system comprises a head-mounted display 100 that overlays digital information onto the user's real-world view. The head-mounted display 100 comprises an eye tracking system 110 that tracks the user's eye movements and gaze direction. The eye tracking system 110 uses the disclosure to estimate the strength of the user's prescription glasses 160, improving the accuracy of the eye tracking and enhancing the user's augmented reality experience.

In conclusion, the disclosure provides a method and system for estimating the strength of prescription glasses for a user of a head-mounted display. The disclosure involves illuminating the user's eyes and prescription glasses, capturing images of the eyes, the prescription glasses, and reflections on the prescription glasses, and determining the power of the lenses of the prescription glasses by analyzing the reflections. The disclosure uses various techniques and technologies, including the base curve rule, a curvature regressor, and machine learning, to accurately estimate the power of the lenses. The disclosure has potential applications in various fields, including virtual reality, augmented reality, and mixed reality.

The terminology used herein is for the purpose of describing particular aspects only and is not intended to be limiting of the disclosure. As used herein, the singular forms "a," "an," and "the" are intended to include the plural forms as well, unless the context clearly indicates otherwise. As used herein, the term "and/or" includes any and all combinations of one or more of the associated listed items. It will be further understood that the terms "comprises," "comprising," "includes," and/or "including" when used herein specify the presence of stated features, integers, actions, steps, operations, elements, and/or components, but do not preclude the presence or addition of one or more other features, integers, actions, steps, operations, elements, components, and/or groups thereof.

It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure.

Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present.

Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein.

It is to be understood that the present disclosure is not limited to the aspects described above and illustrated in the drawings; rather, the skilled person will recognize that many changes and modifications may be made within the scope of the present disclosure and appended examples and claims. In the drawings and specification, there have been disclosed aspects for purposes of illustration only and not for purposes of limitation, the scope of the disclosure being set forth in the following claims.

### Examples:

Example 1. A method for estimating the strength of prescription glasses for a user of a head-mounted display (100), the method comprising:
illuminating user's eyes (150) and prescription glasses (160) using illuminators (130);
capturing images (190) of the eyes (150), the prescription glasses (160), and reflections (180) on the prescription glasses (160) using at least one camera (120); and
determining power of lenses (165) of the prescription glasses (160) by analyzing the reflections.

Example 2. The method according to example 1, further comprising setting camera brightness controlling parameters of the at least one camera (120) to capture the reflections (180) on the prescription glasses (160), camera brightness controlling parameters comprising at least one of exposure time or camera gain.

Example 3. The method according to examples 1 or 2, further determining positions of the reflections (180) in the images (190) and identifying illuminators (130) responsible for each of the reflections (180).

Example 4. The method according to any one of examples 1 to 3, wherein determining the power of the lenses (165) comprises using a correlation between the power of the lenses (165) and a radius of curvature (170) of a surface of lenses (165), wherein the radius of curvature (170) of the lenses (165) is estimated based on the positions of the reflections (180) in the images (190) and the positions of the respective illuminators (130).

Example 5. The method according to example 4, wherein the reflections (180) used to estimate the radius of curvature (170) of the lenses (165) are reflections (180) on an outer surface (168) of the lenses (165).

Example 6. The method according to examples 4 or 5, wherein the correlation is a base curve rule.

Example 7. The method according to examples 4 to 6, further comprising approximating the surface of the lenses (165) as a spherical shape.

Example 8. The method according to any one of examples 1 to 7, further comprising determining power for each of a right lens (167) and a left lens (166) in a pair of the prescription glasses (160).

Example 9. The method according to any one of examples 1 to 3, further comprising estimating the curvature (169, 169a, 169b) of an outer surface (168) and/or an inner surface (178) of the prescription glasses (160) for each of the lenses (165) by utilizing image processing of the captured images (190).

Example 10. The method according to example 9, further comprising utilizing a pre-trained regressor to estimate said curvature (169, 169a, 169b) by a machine learning process.

Example 11. The method according to example 10, wherein the machine learning process comprises at least one of linear regression or non-linear regression.

Example 12. The method according to any one of examples 10 or 11, wherein the pre-trained regressor is trained using annotated features comprising curvatures and reflections (180) from the outer surface (168) and/or the inner surface (178) of the lens from images (190) of users wearing prescription glasses.

Example 13. A head-mounted display (100) comprising:
an eye tracking system (110) configured to determine a gaze direction, and an entrance pupil location of user's eyes (150);
at least one camera (120) configured to capture images (190) of the user's eyes (150) and the prescription glasses (160);
illuminators (130) configured to illuminate the user's eyes (150) and the prescription glasses (160); and
a computer analysis system (140) configured to analyze the captured images (190) and determine power of lenses (165) of the prescription glasses (160) by analyzing reflections (180) on the prescription glasses (160).

Example 14. The head-mounted display (100) according to example 13, wherein the eye tracking system (110) is configured to perform pupil center cornea reflection (PCCR) eye tracking.

Example 15. The head-mounted display (100) according to examples 13 or 14, wherein the illuminators (130) are LED illuminators having a wavelength range of 750nm - 1400nm.

Example 16. The head-mounted display (100) according to any one of examples 13 to 15, wherein the computer analysis system (140) is configured to optimize camera brightness parameters comprising at least one of exposure time or camera gain to capture reflections (180) on an outer surface (168) and/or an inner surface (178) of the prescription glasses (160).

Example 17. The head-mounted display (100) according to any one of examples 13 to 16, wherein the computer analysis system (140) is configured to identify illuminators (130) responsible for each reflection (180) and determine a curvature (169, 169a, 169b) of the lens by analyzing the reflections (180).

Example 18. The head-mounted display (100) according to example 17, wherein the computer analysis system (140) is configured to approximate a radius of curvature (170) and estimate power of the lenses (165) based on the curvature (169, 169a, 169b).

Example 19. The head-mounted display (100) according to any one of examples 17 or 18, wherein the computer analysis system (140) is configured to utilize a pre-trained regressor to estimate the curvature (169, 169a, 169b) of the outer surface (168) and/or the inner surface (178) of the prescription glasses (160) for each of the lenses (165).

## Claims

1. A method for estimating the strength of prescription glasses (160) for a user of a head-mounted display (100), the method comprising:
capturing images (190) of each of the user's eyes (150) and the prescription glasses (160) from different viewpoints;
determining a number of candidate ellipses representing the same iris (156) from the captured images (190);
determining a center (157) and radius (158) of each iris (156) in a 3D space using triangulation of the candidate ellipses; and
analyzing a deviation in the determined radius (158) of each iris (156) to infer the power of the lenses (165) of the prescription glasses (160).

2. The method according to claim 1, wherein determining each ellipse comprises employing edge detection to fit an ellipse for each iris (156).

3. The method according to claim 1, wherein determining each ellipse comprises utilizing an ellipse regressor.

4. The method according to any one of claims 1 to 3, wherein the deviation in the radius (158) of each iris (156) is the estimated difference in size of the radius (158) from a human average iris radius.

5. The method according to claim 4, wherein the human average iris radius is 5.5mm.

6. The method according to any one of claims 1 to 5, wherein determining the power of the lenses (165) is based on a pre-calculated relationship between the power of the lenses (165) of the prescription glasses (160) and the deviation in the determined radius (158) of an iris (156).

7. The method according to claim 6, wherein the pre-calculated relationship is determined through a machine learning process where the estimated iris radius is known prior to refraction through the lenses (165).

8. The method according to claim 7, wherein the machine learning process comprises a regression model.

9. A head-mounted display (100) comprising:
an eye tracking system (110) configured to determine a gaze direction of user's eyes (150);
at least one camera (120) configured to capture images (190) of each of the user's eyes (150) and the prescription glasses (160) from different viewpoints;
and a computer analysis system (140) configured to
determine a number of candidate ellipses representing the same iris (156) from the captured images (190),
determine a center (157) and radius (158) of each iris (156) in a 3D space using triangulation of the candidate ellipses, and
analyze a deviation in the determined radius (158) of each iris (156) to infer the power (171) of the lenses (165) of the prescription glasses (160).

10. The head-mounted display (100) according to claim 9, wherein the computer analysis system (140) is further configured to estimate a difference in size of the radius (158) from a human average iris radius.

11. The head-mounted display (100) according to claim 9, wherein the computer analysis system (140) is further configured to determine the power of the lenses (165) of prescription glasses (160) based on a pre-calculated relationship between the power of the lenses (165) of the prescription glasses (160) and the deviation in the determined radius (158) of each iris (156).

12. The head-mounted display (100) according to claim 11, wherein the pre-calculated relationship is determined through a machine learning process where the estimated iris radius (158) is known prior to refraction through lenses (165).

13. The head-mounted display (100) according to claim 12, wherein the machine learning process comprises a regression model.
